# EUROPEAN PATENT SPECIFICATION

(11) **EP 2 893 032 B1**
(45) Date of publication and mention of the grant of the patent: **17.01.2018**
(21) Application number: 13834474.2
(22) Date of filing: 05.09.2013
(51) Int. Cl.: C12Q 1/37, C07K 14/81, G01N 33/573

(54) **METHODS AND COMPOSITION FOR DETECTING INTESTINAL CELL-BARRIER DYSFUNCTION**
VERFAHREN UND ZUSAMMENSETZUNG ZUM NACHWEIS EINER DYSFUNKTION DER INTESTINALEN BARRIERE
PROCÉDÉS ET COMPOSITION POUR DÉTECTER UN DYSFONCTIONNEMENT DE LA BARRIÈRE DE CELLULES INTESTINALES

(30) Priority: 05.09.2012 US 201261697190 P
(43) Date of publication of application: 15.07.2015
(73) Proprietor: Maximus Diagnostic Technologies LLC, Little Rock, AR 72223 (US)
(72) Inventor: IRVIN, Randall, Thomas, Sherwood Park, Alberta T8H 1E2 (CA); LIU, Julia, Little Rock, AR 72223 (US); DAVIS, Elisabeth, Melika, Little Rock, AR 72211 (US)
(74) Representative: Kaminski Harmann
(86) International application number: PCT/US2013/058296
(87) International publication number: WO 2014/039699

(56) References cited:
- WO-A1-2011/128429
- WO-A2-2008/043566
- US-A1- 2003 040 681
- US-A1- 2005 136 492
- US-A1- 2010 003 196
- US-A1- 2010 222 228
- MCALINDON, M.E. ET AL: "Interleukin 1 beat converting enzyme (ICE) is expressed by macrophages in the Lamina propria of active IBD mucosa.", GUT, vol. 39, 532, 1 January 1996 (1996-01-01), XP002751655,
- BEDNER ELZBIETA ET AL: "Activation of caspases measured in situ by binding of fluorochrome-labeled inhibitors of caspases (FLICA): Correlation with DNA fragmentation", EXPERIMENTAL CELL RESEARCH, ACADEMIC PRESS, US, vol. 259, no. 1, 25 August 2000 (2000-08-25), pages 308-313, XP009149512, ISSN: 0014-4827, DOI: 10.1006/EXCR.2000.4955 [retrieved on 2002-03-25]
- GARCIA-CALVO M ET AL: "Inhibition of human caspases by peptide-based and macromolecular inhibitors", JOURNAL OF BIOLOGICAL CHEMISTRY, AMERICAN SOCIETY FOR BIOCHEMISTRY AND MOLECULAR BIOLOGY, US, vol. 273, no. 49, 4 December 1998 (1998-12-04), pages 32608-32613, XP002222060, ISSN: 0021-9258, DOI: 10.1074/JBC.273.49.32608
- BULLOK KRISTIN E ET AL: "Biochemical and in vivo characterization of a small, membrane-permeant, caspase-activatable far-red fluorescent peptide for imaging apoptosis", BIOCHEMISTRY, AMERICAN CHEMICAL SOCIETY, US, vol. 46, no. 13, 1 April 2007 (2007-04-01) , pages 4055-4065, XP009149527, ISSN: 0006-2960, DOI: 10.1021/BI061959N [retrieved on 2007-03-10]
- Britta Siegmund: "Commentary Interleukin-1b converting enzyme (caspase-1) in intestinal in ammation", , 1 July 2002 (2002-07-01), XP055231977, Retrieved from the Internet: URL:http://ac.els-cdn.com/S000629520201064 X/1-s2.0-S000629520201064X-main.pdf?_tid=a 8957080-94ff-11e5-98b7-00000aab0f27&acdnat =1448626327_ab524a5d3e9710457383aec7d87344 5f [retrieved on 2015-11-27]
- KNODLER ET AL.: 'Dissemination of invasive Salmonella via bacterial-induced extrusion of mucosal epithelia' PROCEEDINGS OF THE NATIONAL ACADEMY OF SCIENCES vol. 107, no. 41, 12 October 2012, pages 17733 - 17738, XP055225762
- LIU ET AL.: 'Mind the Gaps: Confocal Endomicroscopy Showed Increased Density of Small Bowel Epithelial Gaps in Inflammatory Bowel Disease' JOUMAL OF CLINICAL GASTROENTEROLOGY vol. 45, 01 March 2011, pages 240 - 245, XP055225764
- LIU ET AL.: 'Epithelial Cell Extrusion Leads to Breaches in the Intestinal Epithelium' INFLAMMATORY BOWEL DISEASES vol. 19, 18 March 2013, pages 912 - 921, XP055225768
- M E Mcalindon ET AL: "Expression of interleukin 1beta and interleukin 1beta converting enzyme by intestinal macrophages in health and inflammatory bowel disease", GUT, vol. 42, no. 2, 1 February 1998 (1998-02-01), pages 214-219, XP055231969, UK ISSN: 0017-5749, DOI: 10.1136/gut.42.2.214

## Description

### Field of the Invention

The present invention relates methods and a composition for detecting cell-barrier dysfunctions associated with irritable bowel syndrome (IBS) and inflammatory bowel disease (IBD).

### Background of the Invention

Irritable bowel syndrome (IBS) is a common clinical condition characterized by changes in bowel frequency, consistency and abdominal discomfort. Epidemiologic studies using the Rome II criteria indicate that the prevalence of IBS varies from 5% to 12% in North America, 1% to 22% in Asia, and 1 to 8% in Europe. There is a female predominance observed in most studies, particularly from Western countries. One of the main drivers of IBS may be abnormal intestinal epithelial cell (IEC) extrusion.

Inflammatory bowel disease (IBD) is a group of inflammatory conditions of the colon and small intestine. The major types of IBD are Crohn's disease and ulcerative colitis. Both IBS and IBD may be due to, or aggravated by abnormal intestinal epithelial cell (IEC) extrusions that lead to cell-barrier dysfunction in the patient.

### Summary of the Invention

The invention includes, in one embodiment, a method for detecting irritable bowel syndrome (IBS) or inflammatory bowel disease (IBD) in a patient by (a) staining patient intestinal, oropharyngeal, or buccal epithelial cells with a probe having a detectable marker conjugated to a caspase-1 inhibitor, and (b) examining the stained intestinal, oropharyngeal, or buccal epithelial cells for the presence of elevated levels of detectable marker, relative to similarly-stained intestinal, oropharyngeal, or buccal epithelial cells from a normal individual, respectively, as evidence of above-normal levels of caspase-1 associated with the patient intestinal, oropharyngeal, or buccal epithelial cells

Elevated levels of caspase-1 in the patient intestinal epithelial cells (IECs), oropharyngel epithelial cells (OECs), or buccal epithelial cells (BECs) is an indicator of cell barrier dysfunction associated with irritable bowel syndrome (IBS) or inflammatory bowel disease (IBD) in the patient.

In one embodiment, patient IECs are obtained from a biopsy or aspiration from the intestinal lining of the patients, stained *in vitro* with a fluorescence marker, and analyzed for fluorescence level. In another embodiment, patient OECs are obtained from a dental biopsy or aspiration of oropharynx cells in the patient, stained *in vitro* with a fluorescence marker, and analyzed for fluorescence level. In a third embodiment, patient BECs are obtained, e.g., by gentle swabbing of the cheek, stained *in vitro* by a fluorescence marker, and analyzed for fluorescence level. Florescence detection may be by fluorescence microscopy, fluorescence plate readers, flow cytometry, or other methods suitable for detecting and measuring fluorescence levels.

In another general embodiment, elevated levels of caspase-1 in OECs or BECs is diagnostic of Crohn's disease, a major type of IBD.

The probe may be a conjugate of the caspase-1 inhibitor, such as the tetrapeptide YVAD, and a fluorochrome. An exemplary probe has the structure Alexa Fluor 488-GGGG-YVAD-FMK.

In an exemplary embodiment the cells are stained (a) a first probe comprising a first detectable marker conjugated to a caspase-1 inhibitor, and (b) second probe comprising a second detectable marker different from the first marker conjugated to a caspase-3&7 inhibitor. The cells are analyzed to determine the ratio of marker ssociated with caspase-1 to marker relative the marker associated with caspase-3&7. The ratio of caspase-1 to caspase-3&7 markers is significantly lower, e.g., at least 40% lower, in healthy subjects than in subjects with IBS or IBD. An exemplary second probe is a conjugate of Caspase-3/7 Inhibitor I and a fluorochrome whose peak absorption and emission wavelengths are different from those of the first-probe fluorochrome.

The method may be used to indicate patient treatment by a caspase-1 inhibitor, an anti-inflammatory agent, a probiotic or a combination of these agents when the level of caspase-1 in the IECs is significantly elevated above normal levels.

In another general embodiment, the IECs, OECs, or BECs are stained *in situ,* and viewed by probe-based confocal laser endomicroscopy (pCLE).

In other aspect, the invention includes a method of detecting intestinal cell barrier dysfunction in a patient by the steps of obtaining an *in situ* image of a patient's IEC's by probe-based confocal laser endomicroscopy (pCLE), and counting IECs in the image to determine the number of gaps in the imaged IECs. A gap density of greater than about 2 per hundred cells is indicative of cell barrier dysfunction, and may be used as an indicator for patient treatment, e.g., by a probiotic agent.

Also disclosed is a probe composition for use in detecting intestinal cell barrier dysfunction. The composition includes (a) a first probe comprising a first detectable marker conjugated to a caspase-1 inhibitor, and (b) a second probe comprising a second detectable marker different from the first marker conjugated to a caspase-3/7 inhibitor. The first probe may be a conjugate of the tetrapeptide YVAD and a fluorochrome, such as a probe having the structure Alexa Fluor 488-GGGG-YVAD-FMK. The second probe may be a conjugate of Caspase-3/7 Inhibitor I and a fluorochrome different from that in the first probe.

These and other objects and features of the invention will become more fully apparent when the following detailed description of the invention is read in conjunction with the accompanying drawings.

### Brief Description of the Drawings

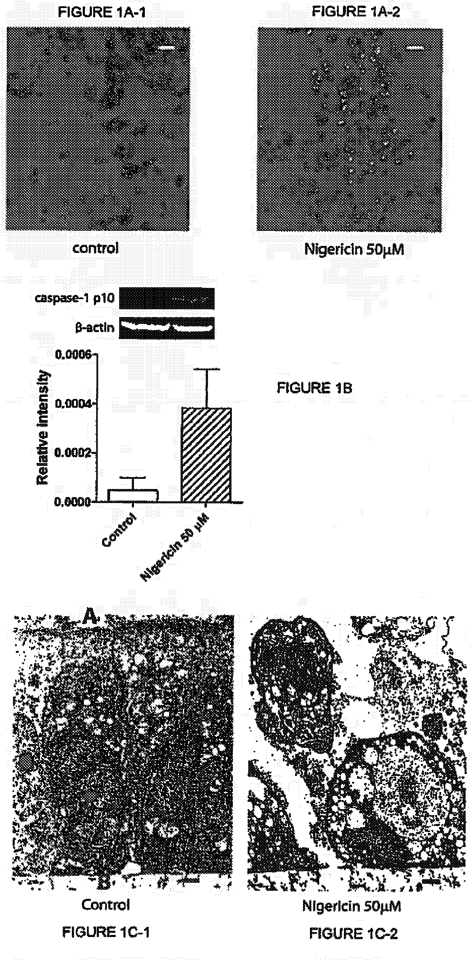

Figure 1. Caspase-1 activation of IECs induced cell extrusion in the polarized T84 monolayer. (a) representative FLICA 1 staining (green) of activated caspase-1 in nigericin treated (50 µM) cultured T84 cells. Red, ZO-1 stain; blue, DAPI, green, FLICA 1 stain (scale bars, 50 µm). (b) increased active caspase-1 (p10) expression in nigericin-treated (50 µM) T84 cells. (c) TEM appearance of T84 cells treated with nigericin: chromatin condensation around the nuclear membrane, small and large clear vacuoles with dense bodies in the cytoplasm, and intact mitochondria with increase of the matrix density. A, apical surface, B, basal surface, N, nucleus (scale bars, 2 µm).

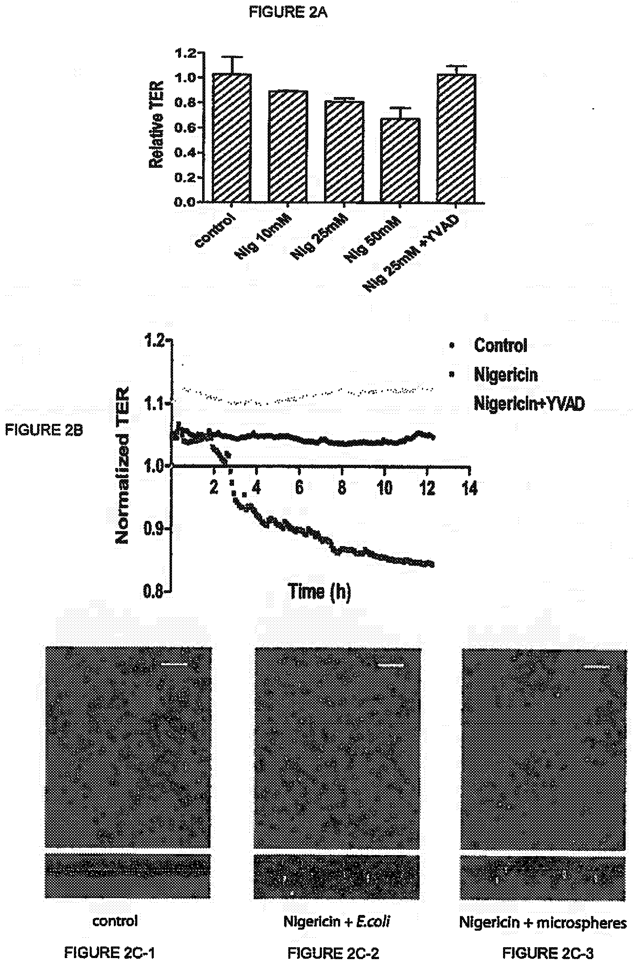

Figure 2. Altered permeability of the polarized monolayers after caspase-1 activation. (a) dose-dependent reduction in TER (±S.D.) of T84 monolayers treated with nigericin, reversed with Ac-YVAD-CMK (at nigericin 25 µM). (b) time-dependent reduction in TER as measured by ECIS of T84 monolayers treated with nigericin, reversed with Ac-YVAD-CMK (at nigericin 25 µM). (c) movements of Fluoresbrite® YG microspheres and *E. coli* TMW2.497 across the monolayer treated with nigericin 10 µM overnight. Red, ZO-1 stain; center image green, 1 µm microspheres, right image green, *E. coli* TMW2.497 (scale bars, 50 µm). Data are representative of three independent experiments.* P<0.05.

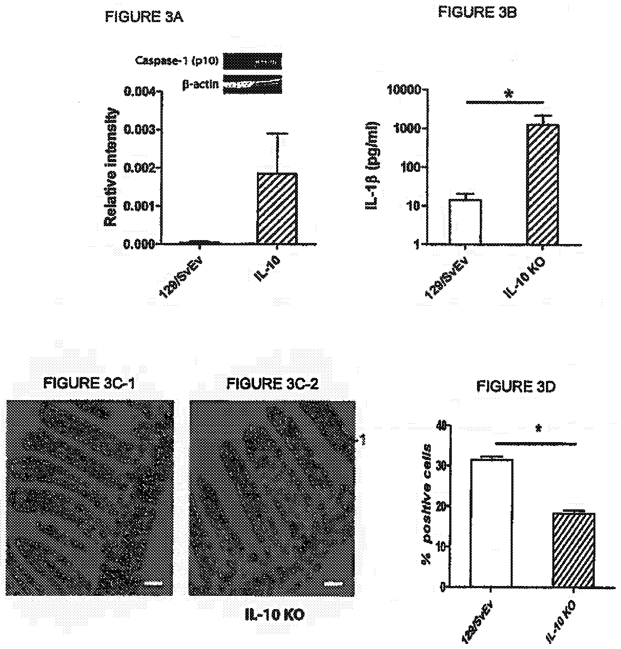

Figure 3. Increased caspase-1 activation in IL-10 KO compared to WT mice. (a) increased active caspase-1 (p10) expression in the IL-10 KO by Western blot analysis. (b) increased active IL-1β in intestinal tissue of the IL-10 KO (N=5). (c) representative images of PCNA stained intestinal sections from WT and IL-10 KO mice (scale bars, 50 µm). (d) number of positive PCNA staining cells per crypt of rodent intestinal tissue. * P<0.05.

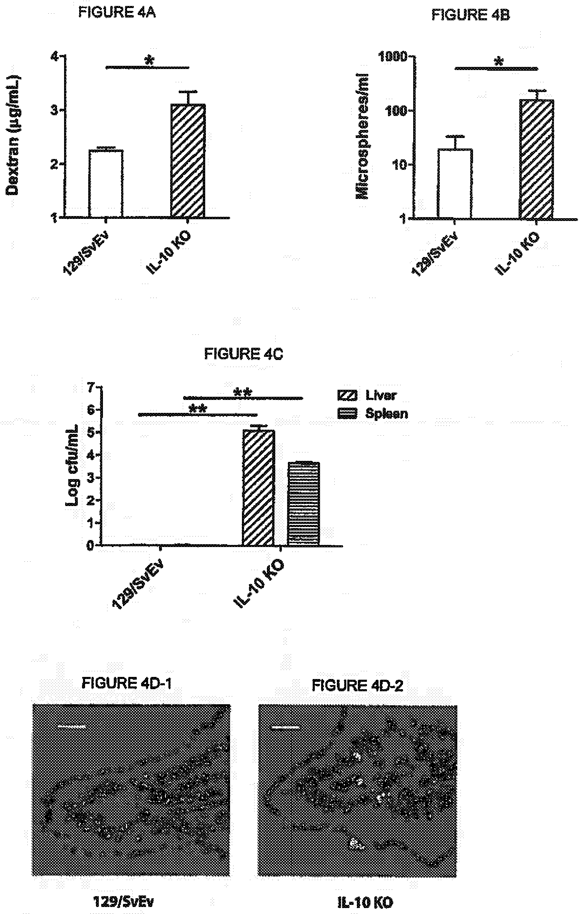

Figure 4. Increased permeability to luminal microparticles and microbes in the IL-10 KO mice. (a) permeation of orally administered FITC-dextran into blood samples (N=4). (b) presence of orally administered 0.5 µm Fluoresbrite® microspheres in blood samples (N=6). (c) translocation of *E*. *coli* TMW2.497 to liver and spleen (N=4). (d) representative images of *E*. *coli* TMW2.497 entering an extrusion zone in the mouse intestine. *P<0.05, ** P<0.01.

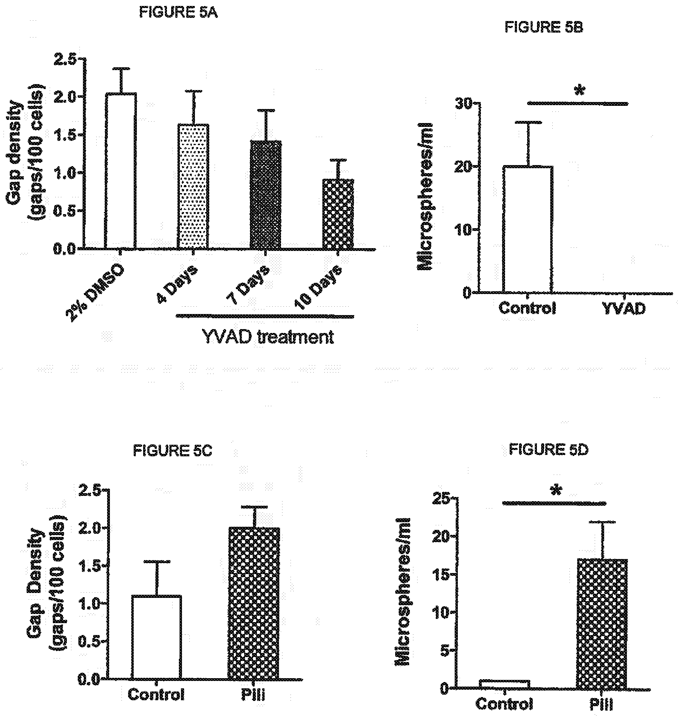

Figure 5. Modulation of caspase-1 on IEC extrusion and permeation of microspheres *in vivo.* (a) treatment with Ac-YVAD-CMK 10 mg/kg on IEC extrusion in IL-10 KO mice as measured by epithelial gap density using confocal endomicroscopy over time (N=5). (b) presence of orally administered 0.5 µm Fluoresbrite® microspheres in the blood samples of IL-10 KO mice (N=6). (c) orogavage of type IV pili of *P*. *aeruginosa* 0.33 mg/kg for 1 day on IEC extrusion in WT mice (N=3) as measured by gap density. * P<0.05.

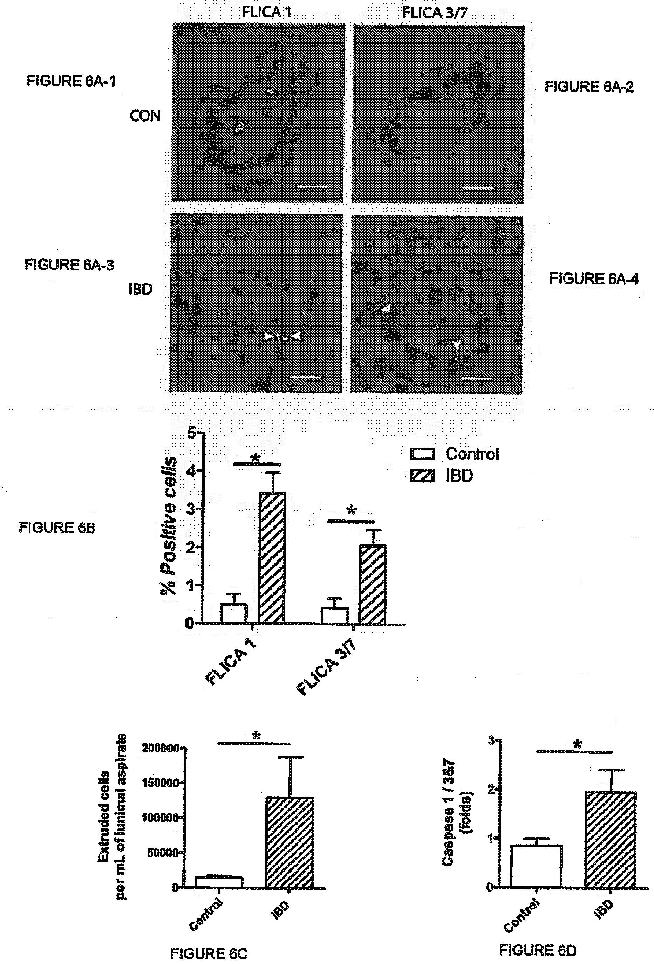

Figure 6. Caspase-1 and caspase-3&7 activation of IECs in patients. (a) representative activated caspase-1 and caspase-3&7 stains of mucosal biopsy samples, white arrowheads indicating positively stained IECs (scale bars, 50 µm). (b) FLICA 1 or 3&7 stained cells normalized to the total number of epithelial cells (±S.D.) in mucosal biopsy samples in control (N=3) and IBD (N=3) patients. (c) representative epithelial and immune cells from cytology block prepared from luminal aspirates of IBD patients (H&E stain, magnification 400X). (d) number of extruded IECs (±S.D.) in luminal aspirates of control (N=7) and IBD (N=11) patients. (e) the ratio of activated caspase-1 over caspase-3&7 positive extruded cells in the luminal aspirates. * P<0.05.

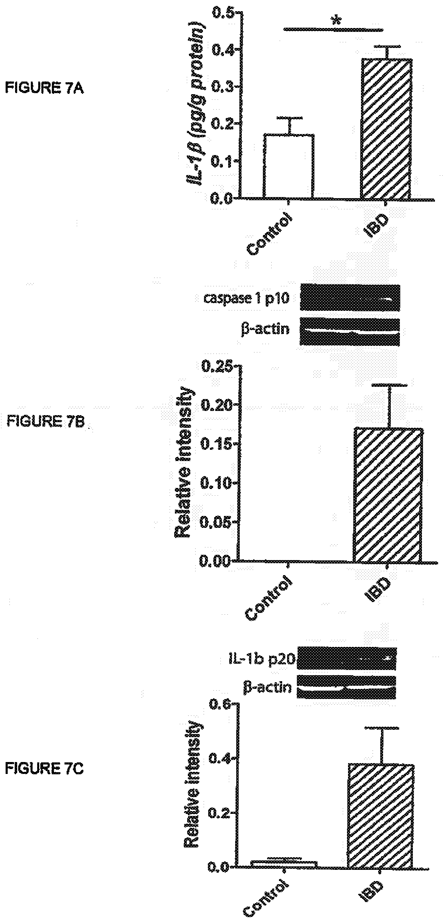

Figure 7. Activated caspase 1 and IL-1β expression in the mucosal tissue of asymptomatic control (N=3) and IBD patients (N=3). (a) increased active IL-1β expression in IBD compared to control patients as measured by ELISA. (b) Western blot analysis confirming increased expression of active IL-1β in terminal ileum of IBD patients. (c) increased active caspase-1 (p10) expression in IBD patients by Western blot analysis. * P<0.05.

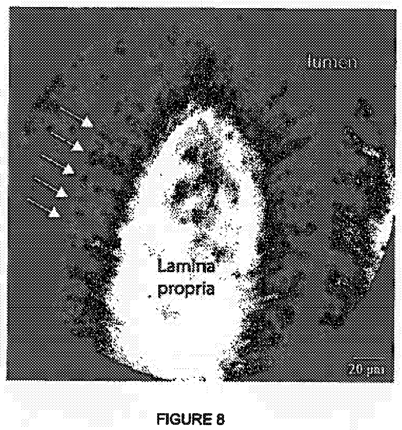

Figure 8. Representative pCLE image of the terminal ileum of from a patient used for counting of epithelial cells and gaps. No gaps were observed in this image. White arrows indicating individual epithelial cells used in the counting of cells.

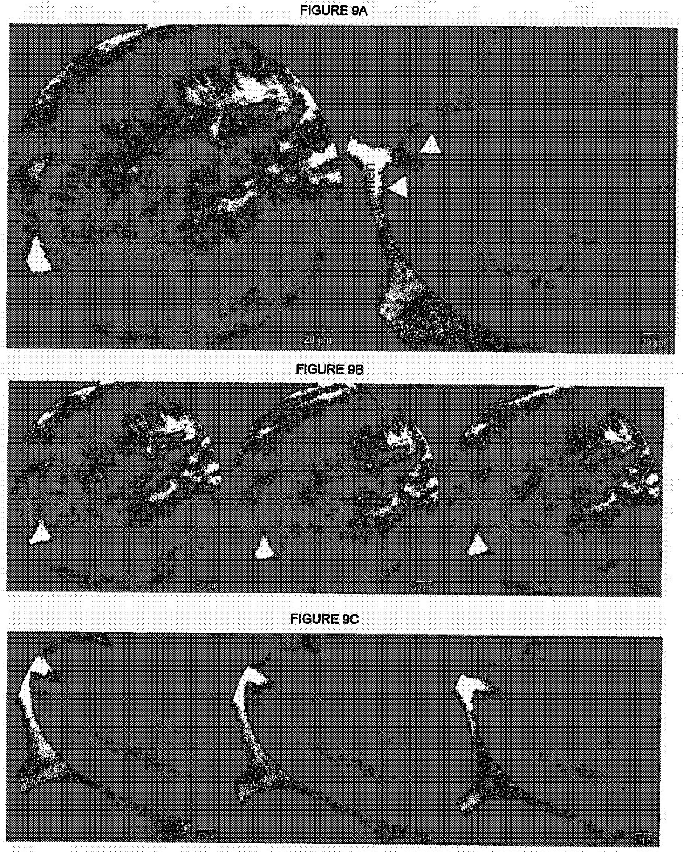

Figure 9. pCLE image of the terminal ileum of patients. a) representative image from the terminal ileum of a healthy control patient (left) and a patient with IBS (right). The lamina propria and lumen of the villi are labeled. White arrow heads indicate two adjacent epithelial gaps which appear as hyperdense areas in the lining of the epithelium. b) Three consecutive pCLE images used in the analysis for the control patient. C) Three consecutive pCLE images used in the analysis for the IBS patient. Scale bar: 20 *µ*m.

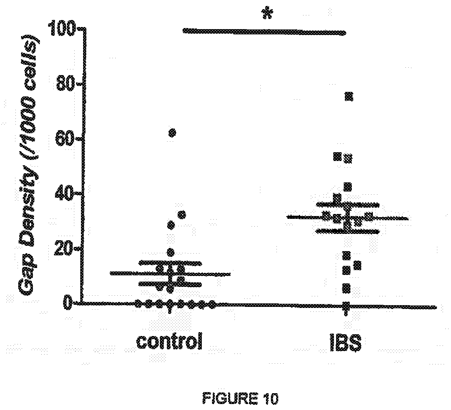

Figure 10. Comparison of the epithelial gap density in the terminal ileum of control and IBS patients (median ± interquartile range). Epithelial gap density is expressed as the number of epithelial gap per 1000 cells counted. * denotes p < 0.001.

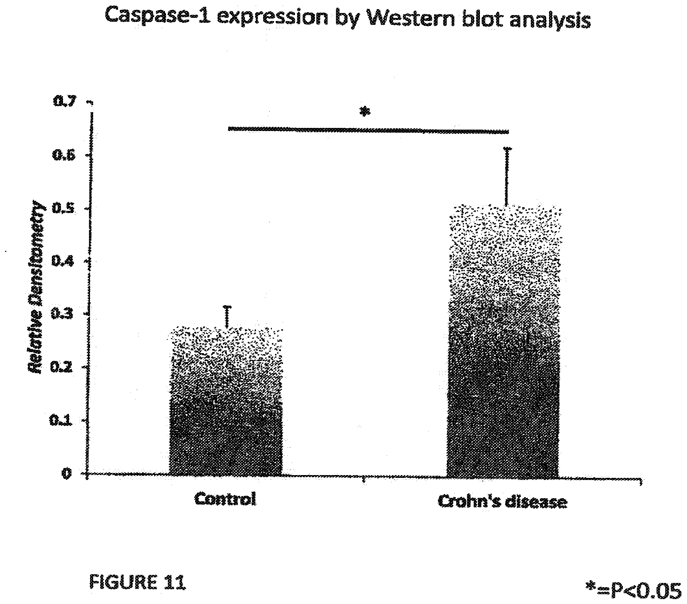

Figure 11 shows levels of Caspase-1 expression, as determined by Western blot analysis, in opharyngeal epithelial cells from a dental biopsy in a normal patient and a Crohn's disease patient.

### Detailed Description of the Invention

### A. Method of Detecting Cell-barrier Dysfunction by Caspase-1 Staining

### A1. Caspase-1 mediated IEC extrusion results in breaches in the epithelial monolayer

To investigate the morphology of caspase-1-induced IEC extrusion, we applied nigericin, a well-established Nlrp3-dependent inflammasome activator to polarized T84 monolayers. Using FLICA 1 staining, we observed increased activated caspase-1 and cell extrusion in monolayers at 3-hours post-treatment (Figure 1a). Active caspase-1 expression in nigericin-treated T84 cells was confirmed by Western blot analysis (Figure 1b). The morphologic appearance of extruded cells from the monolayers by transmission electron microscopy (TEM) revealed distinct chromatin condensation in the nuclei, intact mitochondria and small or large clear vacuoles in the cytoplasm (Figure 1c).

To determine whether this form of cell extrusion results in loss of barrier function, we measured the trans-epithelial electrical resistance (TER). Following nigericin exposure, dose-dependent barrier dysfunction developed, which was abrogated by pretreatment with a selective, potent and irreversible caspase-1 inhibitor Ac-YVAD-CMK at 3 hours (Figure 2a) and after overnight treatment (Figure 2b). Given that the breach in the T84 monolayers appeared to be 1 - 2 µm in diameter on TEM images, we evaluated the epithelial integrity to microparticles (1µm Fluoresbrite® Microspheres) and microbes (*E. coli* TMW2.497) using the lowest dose of nigericin treatment. Movements of microspheres and *E*. *coli* from the upper chamber through the monolayer to the lower chamber of the Transwell were observed (Figure 2c). Fluoresbrite microspheres and *E*. *coli* TMW2.497 were recovered in the media from basolateral well.

### A2. Modulation of caspase-1 on cell extrusion and epithelial integrity in vivo

To understand the effect of caspase-1 induced IEC extrusion on the permeability of the intestine *in vivo,* we first examined whether increased cell extrusion (as measured by increased density of epithelial gaps) observed in the IL-10 KO compared to 129/SvEv (WT) mice was due to increased caspase-1 activation. Increased active caspase-1 expression in the small intestine of IL-10 KO mice was seen on Western blot analysis (Figure 3a) and was confirmed with increased active IL-1β expression by ELISA (Figure 3b). To determine if reduced cellular proliferation in IL-10 KO contributed to the differences in epithelial gap densities observed, we stained the intestinal samples from two mouse strains with PCNA. IL-10 KO had a 38% reduction in cellular proliferation compared to WT mice (Figures 3c and d).

The effect of increased IEC extrusion on intestinal permeability was investigated with permeation of macromolecules (dextran) and microparticles (Fluoresbrite® Microspheres) into the blood, and translocation of microbes (*E*. *coli* TMW2.497) to liver and spleen in the IL-10 KO and WT mice. Increased IEC extrusion correlated with enhanced permeation of dextran (Figure 4a) and 0.5 µm microspheres (Figure 4b) into the blood, and translocation of *E*. *coli* (Figure 4c) as determined by tissue cultures. Confocal microscopy of ileal tissues from mice gavaged with GFP labelled *E. coli* revealed the presence of bacteria near extrusion zones in the IL-10 KO intestine (Figure 4d).

To evaluate the effect of caspase-1 inhibition on IEC extrusion over time *in vivo,* we treated the IL-10 KO mice with a selective caspase-1 inhibitor Ac-YVAD-CMK (10mg/kg) over 4, 7 and 10 day (5 times the mean lifespan of rodent enterocytes⁴⁰) via intraperitoneal injections. The control IL-10 KO group received 10 days of equal volume of 2% (v/v) DMSO. Time-dependent reduction in IEC extrusion as measured by decrease in epithelial gap density resulted (Figure 5a) in the IL-10 KO mice treated with YVAD. The reduction in gap density was accompanied by normalization of permeation of orogavaged 0.5 µm inert latex microspheres into blood at day 7 (Figure 5b).

The effect of caspase-1 activation on IEC extrusion and epithelial integrity was examined with administration of *P. aeruginosa* type IV pili - an ICE-protease activating factor (IPAF) inflammasome activator that could be given orally to induce caspase-1 activation. We chose *P. aeruginosa* type IV pili since nigericin could not be administered orally and was associated with significant systemic toxicity. In WT mice that were oro-gavaged with type IV pili (0.33mg/kg) for one day, we observed a trend towards increased IEC extrusion as measured by higher epithelial gap density compared to control mice gavaged with equal volume of saline (Figure 5c).

### A3. Non-apoptotic IEC extrusion in the human intestine is mediated by caspase-1 activation

To explore whether caspase-1 activation of IECs represents a major mechanism of cell extrusion in humans we collected mucosal biopsies and luminal aspirates from normal-appearing terminal ileum of IBD and asymptomatic control patients. Mucosal biopsy samples were stained with FLICA-1 and 3&7 to identify IECs positive for activated caspase-1 (pyroptotic) or caspase-3&7 (apoptotic) stains (Figure 6a). The ratio of positively stained caspase-1 to caspase-3&7 cells in controls was 1.16:1; which was increased to 1.7:1 in IBD patients (Figures 6b). For analysis of luminal aspirates, control patients had insufficient material for cytology block preparation. In IBD patients, the total number of nucleated cells seen on cytology blocks ranged from 12 to 155 cells, with IECs accounting between 41 to 100% of the cells (Figure 6c). We quantitated the total number of extruded cells in the luminal aspirates collected on the filter significantly higher cell counts were observed in luminal aspirates from IBD patients compared to controls (Figure 6d). The extruded cells and cellular debris were stained with FLICA for activated caspase-1 and 3&7. The images of FLICA stained luminal aspirates were scored based on the intensity of the caspase staining of cells and cellular debris present on the two membranes, similar to a grading scale used for histological samples. Each image was assigned a score of 0 to 4 depending on the intensity of stain and the number of stained cells or cellular debris. Using this scoring system, the ratio of positively stained caspase-1 to caspase-3&7 cells in controls was approximately 1:1, which was increased to 2:1 in IBD patients (Figure 6e).

The expression of active IL-1β in mucosal biopsy samples was measured with ELISA and was significantly higher in IBD patients (Figure 7a). Increased expression of active caspase-1 and IL-1β in mucosal biopsy samples were confirmed with Western blot analysis (Figures 7b and c). Taken together, these results suggest that caspase-1 activation represents a significant mechanism of IEC extrusion in healthy human intestine and appears to be responsible for the majority of increase in cell extrusion observed in IBD patients. In this study, we described an inflammatory form of IEC extrusion mediated by caspase-1 activation that leads to breaches in the epithelium in *vitro and in vivo.* This form of IEC extrusion permitted movement of microparticles and microbes across the polarized monolayers. IEC extrusion in the rodent intestine could be modulated by activation or inhibition of the caspase-1 enzyme. Increased IEC extrusion in the IL-10 KO mice was associated with increased permeation of macromolecules (dextran), microparticles and translocation of commensal bacteria. Modulation of caspase-1 activity *in vivo* resulted in alterations in IEC extrusion with accompanying changes in epithelial integrity as measured by permeation of inert latex microspheres. In patients, caspase-1 mediated IEC shedding could be observed in the small intestine of healthy and IBD patients, with pronounced increase in IBD patients. Our experimental results provide fundamental new insights into the underlying mechanism of IEC extrusion previously reported to compromise epithelial integrity.⁷

Consistent with previous morphologic analysis of duodenal aspirates showing extruded cells with features of pyroptosis and apoptosis, our luminal aspirate studies revealed activation of both caspase-1 and caspase-3&7 in extruded cells. Our mucosal biopsy analysis findings are in agreement with a prior study where apoptosis was found in 44% of shedded IECs using activated caspase-3 staining of the human intestinal specimens. In this study, we observed caspase-3&7 activation in 46% of IECs to be extruded.

Our analysis results of extruded cells and biopsy samples from patient are complementary and consistent, and in agreement with previous studies of extruded IECs. The luminal aspirates analysis may be limited by the fact that extruded IECs can break up into fragments after shedding, therefore, mucosal biopsy analysis results were essential to confirm the relative ratio of caspase-1 and 3&7 positive cells. Since caspase-1 mediated cell extrusion zones may be permeable to microbes, its dramatic rise in IBD patients may contribute to the increased intra-mucosal and lymph node associated bacterial burden observed in previous studies. The barrier function in patients were not examined in the current study. Since the epithelial defects appears to permit the entry of microparticles and microbes, the appropriate test in patients to examine epithelial integrity will require rigorous validation studies. In addition, we have not investigated the closure or healing mechanism of the extrusion zone after caspase-1 mediated cell shedding, which is critical to define the loss of epithelial integrity observed. In apoptosis induced cell extrusion, contraction of surrounding cells and reorganization of the tight junctions are required to prevent the loss of barrier function. Future studies to delineate the biochemical events of the cell shedding process in pyroptosis will facilitate our understandings of the role of tight-junction modifications, contractile proteins involved in extrusion, and the closure mechanism(s) in this form of cell extrusion. A basic understanding of the closure mechanism after caspse-1 mediated cell extrusion may be needed to facilitate the development of a proper test to assess the epithelial integrity in patients.

The morphologic appearance of extruded cells by transmission electron microscopy (TEM) is consistent with previous reports of pyroptotic cells (Figure 1c), and fits the description of the form of IEC extrusion associated with compromised epithelial integrity in humans. The TER study results suggest that breaches in the epithelial lining induced by this form of cell extrusion is caspase-1 dependent. Our data further suggest that cell extrusion zones resulting from caspase-1 activation may provide entry sites for luminal microbes and antigens. Intra-cellular spaces as sites of microbial entry were observed in epithelia undergoing metabolic stress and in a 3 dimensional co-culture system of enterocytes, monocytes and dendritic cells. Here, we observed development of similar barrier defects in the epithelium with inflammasome/caspase-1 activation in IECs alone.

In rodent models, modulation of caspase-1 activity altered IEC extrusion with associated changes in the integrity of the epithelial lining. Compared to apoptosis mediated cell extrusion where barrier function of the epithelium is preserved, we found pyroptosis mediated IEC extrusion introduced breaches in the epithelium that favored microbial and microparticle entry into the mucosa. Induction of pyroptosis with overnight treatment of type IV pili of *P. aeruginosa* resulted in higher IEC extrusion with accompanying increase in permeation of microspheres in the WT mice. Conversely, inhibition of caspase-1 activity in the IL-10 KO mice resulted in a time-dependent reduction in IEC extrusion as measured by epithelial gap density. Based on these observations, we estimated that time to achieve steady state pharmacological activity (5 times the half life) for colitis would be approximately 35-days for the IL-10 KO mice. Therefore, we chose to use permeation of orogavaged latex microspheres - an assessment of epithelial integrity as a surrogate end-point to study the physiologic effect of reduced cell extrusion, rather than the usual clinical end-point - improvement in colitis score. In our study, normalization of permeation of gavaged microspheres was achieved after 7 days of treatment.

Upstream to IL-1β, Nlrp3 is expressed in both immune and epithelial cells, and appears to play an important role in intestinal homeostasis. Nlrp3 -/- mice were more susceptible to experimental colitis induced by DSS, 2,4,6-trinitrobenzene sulfonate (TNBS), or *Citrobacter rodentium* infection. Consistent with previous studies, our results indicate that caspase-1 activation induced IEC extrusion, mediated either via Nlrp3 or other pathways maybe vital to intestinal homeostasis in health. IL-1β and IL-18 production resulting from caspase-1 activation have been shown to contribute to intestinal inflammation in some reports, while more recent studies suggest that caspase-1 conferred protection against colitis and colitis-associated cancer. The discrepancies in experimental results may due in part to the differences in genetic background, gender of the animals used, or variances in the microbial flora of the animal facilities.

In summary, our study results indicate that caspase-1 activation of IECs can induce cell extrusion that contributes to the development of barrier dysfunction in the intestinal epithelium, which may favour microbial entry into the mucosa. This form of cell extrusion appears to be the mechanism responsible for shedding events previously observed to introduce breaches in the epithelial lining.

### A4. Elevated cascase-1 levels in OECs and BECs are diagnostic of Crohn's disease.

To determine whether caspase-1 activation of OECs is diagnostic of Crohn's disease, we obtained dental biopsies of the oropharyngeal region of normal and Crohn's disease individuals, using standard procedures. The biopsied epithelial cells were stained *in vitro* with caspase-1 marker, as above, and examined by fluorescence microscopy to determine caspase-1 levels. As seen from the bar graph in Figure 11, capase-1 levels in Crohn's patients were elevated about twofold over normal levels.

The data demonstrate that assaying caspase-1 levels in humans, by *in vitro* detection of stained OECs, provides a simple method of detecting Crohn's disease. The diagnostic method involving OECs may be performed with BECs, e.g., obtained by a gentle cheek swab, and is also applicable to other IBD and IBS conditions, and may be carried out by *in vivo* staining of OECs or BECs, followed by detection in situ, e.g., using a fluoroscopic tool to determine stained cell fluorescence levels in the oral cavity.

### B. Method of Detecting Cell-barrier Dysfunction by pCLE

A total of 35 patients (17 with IBS and 18 controls) were recruited into the study, one patient thought to have IBS was excluded from further analysis due to the presence of microscopic colitis on colon biopsies. The baseline patient characteristics are shown in Table 1. The mean age for the 16 IBS patients was 42.8 + 18.5 years. There were 7 female and 9 male patients. Control patients (n=18) had a mean age of 47.4 ± 10.1 years, with 10 female and 8 male patients. Indications for colonoscopy in the controls were colorectal cancer screening (n=9) and rectal bleeding or positive fecal occult blood test (n=9). The IBS group included 12 diarrhea-predominant IBS patients and 4 constipation-predominant IBS. For evaluation of other causes of their symptoms, we performed detailed history on all patients to exclude lactose/fructose intolerance. All but one diarrhea predominant IBS patients had serum antibodies (anti-tTG or anti-endomysial antibody) or EGD with biopsy to rule out Celiac disease. The one patient who did not have serology testing or EGD was in a low risk group for Celiac disease. All but two patients had serum TSH checked to rule out thyroid dysfunction as a cause of their symptoms. Normal colonoscopy was the most common endoscopic finding in both IBS and control patients. Other findings were polyps (n=8), diverticulosis (n=4) and hemorrhoids (n=8). Random biopsies of the terminal ileum and colon performed in all IBS patients and controls were within normal limits. Representative pCLE images of control and IBS patients with the three consecutive views used in counting are shown in Figure 2.

IBS patients had significantly higher gap densities compared with controls (Figure 3): the median gap density of IBS patients was 32 (17 to 42) gaps/1000 cells versus 6 (0 to 13) gaps/1000 cells for controls (p<0.001). Since gap density values were not normally distributed (p=0.005, Shapiro-Wilk test), we used median regression analysis to quantify the between-group difference. The estimated median difference in gap density between IBS and controls was 26 (95% CI: 12, 39) gaps/1000 cells. Controlling for age and gender, the median gap density values remained significantly higher in the IBS group relative to the control group (p<0.001), with an estimated median difference of 25 (95% CI: 18, 32) gaps /1000 cells.

We examined the relationships of epithelial gap density with respect to gender, age, and the sub-types of IBS. In control patients, we noted a trend towards negative correlation between epithelial gap density and age, with a Spearman's correlation coefficient (rho) of -0.43 (p=0.07). In addition, we found a trend towards a higher median gap density in females compared to males (11 versus 0 gap/1000 cells, p=0.07). In IBS patients, these trends were not observed. With respect to the sub-types of IBS, patients with diarrhea-predominant IBS (n=12) had a similar median gap density compared to constipation-predominant IBS patients (n=4): 32 versus 38 gaps/1000 cells, respectively.

The estimated 90^{th} percentile of gap density values from the healthy control group was 30 gaps/1000 cells. Using 30 gaps/1000 cells as the cut off for an abnormal gap density, the diagnostic sensitivity of gap density for IBS is 62%, the specificity is 89%, with a positive predictive value of 83%, and a negative predictive value of 73%. The diagnostic accuracy of gap density for IBS is shown in Table 2.

In this study, we found that IBS patients had significantly higher density of epithelial gaps in the terminal ileum as measured by pCLE compared to healthy controls. This finding suggest that elevated epithelial gaps in the intestine of IBS patients, a surrogate marker for increased epithelial cell extrusion in the small bowel, may contribute to barrier dysfunction and low grade mucosal inflammation previously reported in IBS. Although our results are based on a small number of patients, it does provide potential mechanistic insights into the pathogenesis of disease.

There is growing evidence indicating increased intestinal permeability in IBS is associated with alterations in the epithelial tight junctions and changes in cytokine profiles. Altered expression and cellular distribution of the tight junction proteins, including claudin-1 and occludin have been reported in IBS patients. Changes in cytokine profiles further support the notion of enhanced intestinal permeability in IBS patients. The findings of our study indicate that increased epithelial cell extrusion may be a potential mechanism for the barrier dysfunction and mucosal inflammation observed in IBS patients.

In our secondary analysis, we found that female control patients had a trend towards a higher gap density than males. This finding may provide a potential explanation for the higher prevalence of IBS in females. With higher epithelial gaps at baseline, females are more susceptible to the development of the disease. Furthermore, we observed a trend in healthy controls of a negative correlation of gap density with age, which has not been previously reported. These findings should be further investigated in larger studies. We did not observe a difference in epithelial gap density between diarrhea-predominant or constipation-predominant IBS. However, there were only four patients with constipation-predominant IBS included in this study. Significant changes in intestinal permeability of diarrhea-predominant IBS patients have been previously reported, and not constipation-predominant IBS patients.

To date, there are no specific endoscopic findings that can discriminate IBS from healthy patients. Currently, up to 50% of IBS patients undergo colonoscopy during their assessment, with 25% of colonoscopies performed in the United States for IBS - related symptoms. Most colonoscopies are performed to rule out other etiologies of diarrhea, such as microscopic colitis. In our study, using pCLE during routine colonoscopy to localize and quantitate epithelial gaps in the small intestine of IBS and healthy control patients, we were able to demonstrate that IBS patients have a significantly higher density of epithelial gaps. Our findings of increased epithelial gaps in the small intestine not only provide a potential mechanism of pathogenesis of IBS, but also a possible endoscopic criteria for the diagnosis of the disease. In this study, an elevated gap density had a sensitivity of 62% and specificity of 89% for the diagnosis of IBS. As our understanding of IBS pathogenesis evolves, pCLE may be another diagnostic test that can further define this complex group of diseases. Although the gap density is significantly higher in IBS patients compared to controls in our current study, the increase in gap density is much lower compared to IBD patients in our previous report. A comparison of gap densities in control, IBS and IBD patients is shown in supplementary Figure 1.

There are a number of limitations to our study. This is a small study of 34 patients in a single tertiary referral center with expertise in confocal laser endomicroscopy and in the quantification of epithelial gaps. The IBS patients in our study represent a heterogeneous group of patients. We did not restrict the study subjects to diarrhea - predominant or constipation-predominant IBS patients. The goal of the study was to identify any differences in the gap density between IBS and control patients. There could have been errors in the quantification of epithelial gaps and cells using pCLE images. However, since the reviewers were blinded to the indications for the procedures, these errors should be equally distributed between IBS and control patients. Future large, multi-centered studies are needed to confirm the preliminary findings of our current study. In this study, we only imaged the small intestine with pCLE to quantitate epithelial gap density. We have previously performed a validation study characterizing the inter-observer and intra-observer variability of epithelial gap density of the terminal ileum using rodent models. We are not aware of such validation studies for CLE imaging of the colon.

In conclusion, we have shown that the epithelial gap density of the terminal ileum, as determined by pCLE during colonoscopy, is significantly higher in IBS patients than healthy controls. This finding suggests that increased epithelial cell extrusion, as measured by epithelial gap density, may represent a potential mechanism for altered intestinal permeability observed in IBS patients.

### C1. Experimental: Caspase-1 Methods

### Mice

IL-10 KO mice (Jackson Laboratories, Bar Harbor, Maine) and the background 129/SvEv mice (Taconic Farms Inc. Cambridge City, Indiana) bred in our animal facilites for at least 10 generations between 24 to 28 weeks old were used for all experiments. Mice were housed in conventional housing facility with light and dark cycles. The animal protocol was approved by the Animal Care and Use Committee for Health Sciences at the University of Alberta.

### Patient samples

The study protocol was reviewed and approved by the Human Ethics Research Review Board at the University of Alberta, and the study was registered at ClinicalTrial.Gov (NCT00988273). Patients undergoing colonoscopy provided written informed consent to participate in the study. In IBD (N=11, 6 Crohn's disease, 5 ulcerative colitis) and asymptomatic control (N=8) patients undergoing colonoscopy, luminal aspirates from normal appearing terminal ileum were collected after gentle washing of the intestinal surface with 0.9% NaCl solution using a previously described method⁷ and were analyzed immediately (<15 minutes). Cytology blocks were prepared from 25mL of luminal aspirates collected after saline wash, and stained with hematoxlin and eosin for morphologic identification of epithelial or immune cells. For FLICA staining, cells from 5 mL of aspirate fluid were immobilized onto a 25mm polycarbonate Membra-fil Nucleopore membrane with 5.0 uM pore size (Whatman, GE Healthcare Life Sciences, Piscataway, NJ) using vacuum filtration and washed by the filtration of an additional 20mL of PBS (pH 7.4) containing 0.5% (w/v) BSA. Fluorescent active site-directed irreversible inhibitors specific activated caspase-1 and caspase-3&7 (Carboxyfluorescein FLICA Apoptosis Detection Kit; Immunochemistry Technologies LLC, Bloomington, MN) were used to stain aspirated cells directly on the Nucleopore membrane. The membrane with immobilized aspirated cells was cut in half and stained with 1:700 dilution of FAM-YVAD-FMK (FLICA-1) stain to detect activated caspase-1 or FAM-DEVD-FMK (FLICA 3&7) stain to detect activated caspase-3&7. Four mucosal biopsy samples from normal-appearing terminal ileum were obtained for analysis (control N=3, IBD N=3), two biopsy samples were placed in liquid nitrogen, and stored at -80°C until use for cytokines assays. Two biopsy samples were embedded in OCT (Tissue-Tek, Torrence, CA), placed in liquid nitrogen and stored at -80°C until sections were prepared.

### Reagents

Nigericin (Invitrogen, Burlington, ON), Ac-YVAD-CMK (Alexis Biochemicals, Farmingdale, NY), varying diameters (0.5 to 6µm) of Fluoresbrite™ Yellow Green Carboxylate Microspheres (Polysciences Inc, Warrington, PA) were purchased. Type IV pili were prepared from *Pseudomonas aeruginosa* strain K with a method previously described, characterized in terms of purity via SDS-PAGE, ability to bind to asialo-GM1 but not to GM1, and ability to bind to stainless steel. The pili preparation contained low amounts of *P. aeruginosa* serotype 05 LPS that was not detectable on silver stained SDS-PAGE gels. *Escherichia coli* TMW2.497 was an *E. coli* JM109 derivative carrying the gene coding for green fluorescent protein (GFP) on plasmid pQBI-63 were courtesy of Dr. M. Gantzle.

### Cell culture and measurement of in vitro permeability

T84 human colon cancer epithelial cells were maintained in tissue culture plates (10 cm) in Dulbecco's minimal essential medium (DMEM)/F-12, 10% (v/v) heat-inactivated fetal bovine serum (FBS), 1%(w/v) penicillin-streptomycin. The cells were plated onto Transwells (2 × 10⁵ cells/well, 6.5 mm diameter; 0.4 µm-pore size; Coming Life Sciences, Tewksbury, MA) and grown until development of apical junctional complexes (as indicated by a transepithelial resistance of> 2,000 Ω·cm²) for studies. For caspase-1 inhibition experiment, prior to nigericin treatment, the tissue culture medium was removed and fresh medium with 50□M caspase-1 inhibitor (Ac-YVAD-CMK) was introduced. Nigericin (10, 25, 50□M) was added to both the apical and basolateral aspect of the Transwell. Transepithelial resistance (TER) was measured at before and 3h after Nigericin treatment, using a Millicell-ERS Voltmeter and chopstick electrodes (Millipore, Bedford, MA). For microspheres and *E*. *coli* experiments, after overnight incubation with nigericin, 10⁷/ml of 1µm Microspheres or 10⁹/ml *E*. *coli* TMW2.497 were added to the apical aspect of Transwell. One hour after incubation with the microspheres or *E*. *coli,* the cells were fixed in cold methanol for 5 minutes. Cells were then permeabilized in 0.2%(v/v) Triton X-100 for 15 min and blocked for one hour in PBS with 0.2% (v/v) goat serum and 1%(w/v) BSA.

### Protein extraction

Human biopsy samples and rodent ileal tissues were homogenized in lysis buffer (0.01M PBS, 0.5% (v/v) Tween 20, and Halt protease inhibitor (containing dimethyl sulfoxide and 4-(2-aminoethyl)-benzenesulfonyl fluoride, Thermo Scientific, Pittsburgh, PA) on ice for protein extraction. Protein-containing supernatant was separated by centrifugation at 13,000g for 30min at 4°C and stored at -70°C until analysis.

### Cytokine expression assays

Concentration of active IL-1β from human samples was measured with Human IL-1β Ultra-Sensitive Kit (Meso Scale Discovery, Gaithersburg, MD). Active IL-1β expression in mouse intestinal tissue was measured with Mouse Proinflammatory 7-Plex Ultra-Sensitive Kit (Meso Scale Discovery, Gaithersburg, MD). Resulting cytokines were normalized for the total protein content of each individual sample as determined by bicinchoninic acid assay (Pierce, Rockford, IL).

### Western Blot Analysis

Human biopsy tissues, mouse ileal mucosal scrapings and T84 cells were lysed in M-PER Mammalian Protein Extraction Reagent (Thermo Scientific, Pittsburgh, PA) containing protease inhibitors. Total cellular lysates (50µg protein normalized for the samples) were loaded in 15% SDS-PAGE gel and underwent subsequent electrophoretic transfer of proteins to a nitrocellulose membrane. Membranes were blocked with ODYSSEY blocking Buffer (Infrared Imaging System, Marysville, OH) for 1 hour at room temperature (RT) and probed overnight at 4°C with IL-1β antibody (Cell Signaling Technology, Danvers, MA) or caspase-1 antibody (Abcam, Cambridge, MA) with β-actin antibody serving as a loading control (Cell Signaling Technology, Danvers, MA). After washing, membranes were incubated with the fluorescent secondary antibodies for 1h at RT and analyzed by the LI-COR Odyssey* (Infrared Imaging System, Marysville, OH).

### Immunofluorescence analysis of cell culture and intestinal samples

Cell culture samples from caspase-1 activation and permeability experiments were fixed in cold methanol for 5 minutes, incubated with the primary rabbit anti-ZO-1 antibody (Invitrogen, Burlington, ON) overnight at 4°C. After washing, the cells were incubated with either 1:150 dilution of FLICA-1 stain for caspase-1 activation or goat anti-rabbit IgG Alex546 antibody (Invitrogen, Burlington, ON) and counterstained with DAPI. Membranes supporting the monolayers were then excised and mounted onto glass slides (DakoCytomation Mounting Medium, Carpentaria, CA). Frozen human biopsy samples were sectioned at 5-µm, air dried, and acetone-fixed before staining with 1:50 dilution of FLICA-1 for activated caspases-1, and 1:50 dilution of FLICA 3&7 for activated caspase-3&7 (Immunochemistry Technologies LLC, Bloomington, MN). Sections were then post-fixed with 4% paraformaldehyde for 15 min at RT and stained with Rhodamine-phalloidin (Invitrogen, Burlington, ON) for F-actin and DAPI for nuclei.

Rodent intestinal frozen tissue blocks were sectioned at 5 µm using cryostat, placed in RT for 30 minutes, fixed in 4% paraformaldehyde freshly prepared in PBS for 30 minutes. The slides were washed with PBS at 10min, blocked with 2% goat serum and 1% BSA in PBS for 1 hour at RT, permeabilized in 0.2% Triton-X100 in 2% goat serum and 1% BSA in PBS for 30 min. slides were stained by incubation with Alexa568 coupled phalloidin diluted 1:40 in PBS for one hour, excess fluorochrome removed by 3 X15 min rinse with 50 ml PBS, counterstained with DAPI. The slides were mounted for microscopy examination using FluorSave reagent (Calbiochem) as mounting medium.

### Proliferating cell nuclear antigen (PCNA) stain

The mouse terminal ileum tissue were stained with rabbit anti-PCNA antibody (AbCam, Cambridge, MA) using a previously published method. After staining for PCNA, the sections were stained with DAPI and imaged with Zeiss inverted microscope (Zeiss, Toronto, Ontario). PCNA-positive cells were counted by two blinded reviewers in a minimum of 5 villi per animal.

### In vivo permeability assays

*In vivo* permeability was assessed with permeation of FITC-dextran, fluorescent microspheres and bacterial translocation studies. For dextran studies, after an overnight fast with free access to water, mice were gavaged with 0.6 µg/kg FITC-dextran (FD-4, 4 kD; Sigma Aldrich, St. Louis, MO). Blood samples were collected at 4 hours after cardiac puncture, serum was centrifuged at 1,957 x g in 4°C for 20 minutes. Fluorescence emission of the supernatant was measured using 488 nm laser on the Typhoon Variable Mode Imager (GE Healthcare, Piscataway, NJ).

For microsphere studies, mice were gavaged with a mixture containing 10⁷ Fluoresbrite® YG Microspheres with diameter of 0.5, 1.0, 2.0, 3.0, and 6.0 µm in 200 µl solution as previously described after an overnight fast. Blood samples were collected 4 hours post-administration of the beads. Whole blood mixture was then centrifuged at 1,250 X g in pre-heparinized tubes for 10 min at RT, the plasma portion of the samples were removed and centrifuged at 1,250 X g for 5 min before flow cytometry analysis. The remaining buffy coat and hematocrit of the samples were lysed with 5mL of lysing buffer (4.15g NH₄Cl, 0.84g NaHCO₃, 1ml 0.5 mM EDTA at pH 8, and 500mL of ddH₂O) at RT, mixed and centrifuged at 1,250 X g for 5 min at 4 °C X 3. The supernatant was discarded. The WBC pellet was re-suspended in 400 µl of 0.03% PBS with Fetal Bovine Albumin. The plasma and WBC pellet samples were analyzed with flow cytometry for determination of microsphere counts.

For bacterial translocation studies, mice were gavaged with 1x10¹⁰ CFU of GFP-labeled *E. coli* suspended in 0.17 mL of LB broth. After 20 hours, spleen and liver samples were collected under sterile surgical conditions. The organs were suspended in pre-weighed tubes with LB broth, homogenized with sterile RNAase-free plastic pestles for 5-10 minutes. The homogenate was centrifuged, and the supernatant was plated onto four plates at varying dilutions for culture.

### Confocal laser ndomicroscopy and confocal microscopy

Confocal laser endomicroscopy of the mouse ileum and confocal microscopy of whole mount mouse intestinal tissues for determination of epithelial gap density were performed using previously described methods. Cell culture, human and mouse intestinal slides were imaged using a spinning disc confocal microscope (Quorum Technologies Inc, Guelph, ON) using previously described methods.

### Electron Microscopy

Control and nigericin treated T84 cells were fixed with 2%(v/v) glutaraldehyde buffered with 0.1M cacodylate-HCI at pH 7.4 overnight 4°C. After fixation, they were washed in cacodylate buffer and postfixed for 2 h in 1%(w/v) osmium tetroxide, then rewashed in cacodylate buffer. After dehydration in a graded series of ethanol concentrations, specimens were placed in several washes of propylene oxide, and subsequently embedded in Epoxy resin (EPON 12). Ultrathin sections were contrasted with uranyl acetate and lead citrate, and examined with a Hitashi 7650 transmission electron microscope at an accelerating voltage of 60 kV. Fields of view were recorded and printed at final magnifications between 1000 and 4800, calibrated with the aid of carbon-grating replicas.

### Statistical Analyses

Wilcoxon rank sum test computed by GraphPad (La Jolla, CA) Prism 4 was used to compare the samples. Two-sided P-values of less than 0.05 were considered to be significant. Bonferroni adjustments were made for multiple comparisons.

### C2. Experimental: IEC Gap Methods

### Methods

This was a prospective cohort study registered at ClinicalTrial.Gov (NCT00988273). The study protocol was reviewed and approved by the Human Ethics Research Review Board at the University of Alberta. The study group consisted of patients with symptoms consistent with IBS based on the Rome III criteria. The control group consisted of patients undergoing colonoscopy for other indications without symptoms of IBS, most commonly colorectal cancer screening and positive fecal occult testing. The inclusion criteria for the study were: patients over the age of 18 years and ability to give informed written consent. Exclusion criteria included: known allergies to fluorescein or shellfish, impaired renal function (serum creatinine over 1.5 mg/dL), and pregnant or nursing patients. All patients gave written informed consent to participate in the study. Patient demographics, history, physical examination findings, and endoscopic findings were recorded in a prospective database.

We performed standard colonoscopy with intubation of the terminal ileum in all patients. Patients had standard cardiopulmonary monitoring and received intravenous sedation with midazolam and fentanyl. An antispasmodic agent (glucagon) was used as needed to reduce peristalsis and movement artifacts. After intubation of the terminal ileum, 5 mL of 10% fluorescein solution was administered intravenously. Confocal images of the terminal ileum were obtained with the ultra-high-definition probe-based confocal laser endomicroscopy (pCLE) probe (UHD Coloflex, Mauna Kea Technologies, Paris, France) following a previously reported protocol. Frame-by-frame confocal images of the terminal ileum at about 10 cm proximal to the ileocecal valve were collected and digitally stored for analysis. A minimum of five different sites in the terminal ileum were imaged using pCLE. The pCLE imaging usually commenced at 10 cm proximal from the ileo-cecal valve, with subsequent sampling of the 5 to 10 sites from the intestinal surfaces at between 5 to 10 cm proximal to the initial site of imaging. Continuous recordings of the pCLE image videos were made for approximately 10 minutes in all patients, with over 4000 images recorded per patient. Although the endoscopist performing the pCLE was not blinded to the status of the patient, the reviewers of the pCLE images were blinded to the status of the patient and the indication for colonoscopy to minimize bias.

Review and analysis of pCLE images were conducted in a post-hoc manner as previously described. Adequately imaged villi is defined as villi with over 75% surface area visualized in the pCLE images, with a minimum of three consecutive views of the villi seen are selected analysis of epithelial cells and gaps. Of these villi images, epithelial cell and gaps in the villi which had the highest frequency of gaps seen (range: 3 to 10 villi per patient) for any individual patient were counted. A representative image of a counted villi counted is shown in Figure 1. Epithelial cells and gaps were manually counted in villi and the highest frequency of epithelial gaps for any individual patient was used to determine the gap density (range: 3 to 10 villi evaluated per patient). The gap density was calculated as the number of epithelial gaps per 1000 epithelial cells counted in the adequately imaged villi.

The primary study end-point was the cohort comparison of epithelial gap densities as determined by pCLE in the IBS and control patients. We also performed exploratory analysis to examine the relationships between epithelial gap density and gender, age, and the subtypes of IBS (IBS-diarrhea predominant vs. IBS-constipation predominant).

### Statistical Analysis

### Sample size calculation:

The sample size calculation was performed based on the epithelial gap density data of asymptomatic and IBS patients from our previous study.²⁴ Assuming a difference in the mean gap density of 10 gap/1000 cells and a standard deviation of 10 gap/1000 cells, a total of 32 patients (16 per group) would be required to achieve 80% power with type I error (α) of 0.05. Since nonparametric methods were anticipated to be employed, patient enrollment was increased by approximately 10%, for a total of 35 patients.

The primary end-point of the study was epithelial gap density, with the comparison between control and IBS patients conducted using the Wilcoxon rank-sum test. Continuous variables that were normally distributed were expressed as mean ± standard deviation, while non-normally distributed continuous variables were expressed as median (interquartile range). The Shapiro-Wilk test was used to assess the normality of the distribution of epithelial gap density. Further analyses employed nonparametric methods, including the Wilcoxon rank-sum test, Spearman correlation, and median regression. For the primary analysis, two-sided P-values of less than 0.05 were considered to be significant. All analyses were conducted using the STATA data analysis and statistical software (StataCorp LP, College Station, Texas).

Although the invention has been described with respect to specific aspects, embodiments, and applications, it will be appreciated that various changes and modification may be made without departing from the invention as claimed

## Claims

1. An *in vitro* method for detecting irritable bowel syndrome (IBS) or inflammatory bowel disease (IBD) in a patient comprising
(a) staining patient intestinal, oropharyngeal, or buccal epithelial cells with a probe having a detectable marker conjugated to a caspase-1 inhibitor, in particular wherein the detectable marker is fluorescent, and
(b) examining the stained intestinal, oropharyngeal, or buccal epithelial cells, in particular by fluorescence microscopy, a fluorescence plate reader, or fluorescence flow cytometry, for the presence of elevated levels of detectable marker, relative to similarly-stained intestinal, oropharyngeal, or buccal epithelial cells from a normal individual, respectively, as evidence of above-normal levels of caspase-1 associated with the patient intestinal, oropharyngeal, or buccal epithelial cells,
(c) where elevated levels of caspase-1 in the patient intestinal, oropharyngeal, or buccal epithelial cells is an indicator of cell barrier dysfunction associated with irritable bowel syndrome (IBS) or inflammatory bowel disease (IBD) in the patient.

2. The *in vitro* method of claim 1, wherein said staining is conducted on
- (i) patient intestinal epithelial cells obtained from the patient by biopsy or aspiration, or
- (i) oropharyngeal epithelial cells obtained from the patient by a dental biopsy, or
- (i) buccal epithelial cells obtained from a cheek swab of the patient.

3. The *in vitro* method of claim 1, wherein the probe is a conjugate of the tetrapeptide YVAD and a fluorochrome, in particular wherein the probe has the structure Alexa Fluor 488-GGGG-YVAD-FMK.

4. The *in vitro* method of claim of claim 1, which further includes staining the intestinal, oropharyngeal, or buccal epithelial cells with a second detectable probe specific for caspase-3&7, and determining the ratio of marker associated with caspase-1 to marker associated with caspase-3&7, in particular wherein the second probe is a conjugate of Capase3/7 Inhibitor I and a flurochrome.

5. The *in vitro* method of claim 4, wherein the ratio of caspase-1 to caspase-3&7 markers is significantly lower, in particular at least about 40% lower, in healthy subjects than in subjects with IBS or IBD.

6. The *in vitro* method of claim 1, wherein an elevated level of caspase-1 is used as an indicator for patient treatment by a caspase-1 inhibitor or an anti-inflammatory agent.

## Patentansprüche

1. *In vitro*-Verfahren zum Nachweis von Reizdarmsyndrom (IBS) oder einer entzündlichen Darmerkrankung (IBD) bei einem Patienten, umfassend die Schritte:
(a) Anfärben von Darm-, Oropharynx- oder Mund-Epithelzellen des Patienten mit einer Sonde, die einen nachweisbaren Marker aufweist, der mit einem Caspase-1-Inhibitor konjugiert ist, wobei der nachweisbare Marker insbesondere fluoreszierend ist, und
(b) Untersuchen der angefärbten Darm-, Oropharynx- oder Mund-Epithelzellen, insbesondere durch Fluoreszenzmikroskopie, einen Fluoreszenz-Plattenleser oder Fluoreszenz-Durchflusszytometrie, auf das Vorliegen erhöhter Spiegel von nachweisbarem Marker im Verhältnis zu gleichartig angefärbten Darm-, Oropharynx- oder Mund-Epithelzellen von einem normalen Individuum, zum Nachweis von über dem Normalwert liegenden Spiegeln von mit den Darm-, Oropharynx- oder Mund-Epithelzellen des Patienten assoziierter Caspase-1,
(c) wobei erhöhte Spiegel von Caspase-1 in den Darm-, Oropharynx- oder Mund-Epithelzellen des Patienten ein Indikator für eine mit dem Reizdarmsyndrom (IBS) oder einer entzündlichen Darmerkrankung (IBD) des Patienten zusammenhängende Zellbarrieren-Dysfunktion sind.

2. *In vitro-*Verfahren nach Anspruch 1, wobei das Anfärben durchgeführt wird an
- (i) Darm-Epithelzellen des Patienten, die durch Biopsie oder Aspiration von dem Patienten erhalten wurden, oder
- (i) Oropharynx-Epithelzellen, die durch Dentalbiopsie von dem Patienten erhalten wurden, oder
- (i) Mund-Epithelzellen, die durch einen Wangenabstrich des Patienten erhalten wurden.

3. *In vitro*-Verfahren nach Anspruch 1, wobei die Sonde ein Konjugat des Tetrapeptids YVAD und eines Fluorochroms ist, wobei die Sonde insbesondere die Struktur Alexa Fluor 488-GGGG-YVAD-FMK aufweist.

4. *In vitro*-Verfahren nach Anspruch 1, ferner umfassend das Anfärben der Darm-, Oropharynx- oder Mund-Epithelzellen mit einer zweiten nachweisbaren Sonde, die spezifisch für Caspase-3&7 ist, und das Bestimmen des Verhältnisses von mit Caspase-1 assoziiertem Marker zu mit Caspase-3&7 assoziiertem Marker, wobei die zweite Sonde insbesondere ein Konjugat von Capase3/7-Inhibitor I und einem Fluorochrom ist.

5. *In vitro*-Verfahren nach Anspruch 4, wobei das Verhältnis von Caspase-1- zu Caspase-3&7-Markern in gesunden Individuen deutlich niedriger, insbesondere mindestens etwa 40% niedriger ist als in Individuen mit IBS oder IBD.

6. *In vitro*-Verfahren nach Anspruch 1, wobei ein erhöhter Caspase-1-Spiegel als Indikator für eine Behandlung des Patienten mit einem Caspase-1-Inhibitor oder einem entzündungshemmenden Agens genutzt wird.

## Revendications

1. Méthode *in vitro* pour la détection du syndrome du côlon irritable (SCI) ou des maladies inflammatoires intestinales (MII) chez un patient, comprenant :
(a) la coloration de cellules épithéliales intestinales, oropharyngées ou buccales du patient avec une sonde portant un marqueur détectable conjugué à un inhibiteur de la caspase 1, en particulier un marqueur détectable fluorescent, et
(b) l'examen des cellules épithéliales intestinales, oropharyngées ou buccales colorées par microscopie à fluorescence, avec un lecteur de plaques à fluorescence ou par cytométrie de flux à fluorescence pour rechercher la présence de taux élevés du marqueur détectable par rapport à des cellules épithéliales intestinales, oropharyngées ou buccales colorées de la même manière d'un individu sain, respectivement, afin de démontrer la présence de taux de caspase 1 supérieurs à la normale associés aux cellules épithéliales intestinales, oropharyngées ou buccales du patient,
(c) un taux élevé de caspase 1 dans les cellules épithéliales intestinales, oropharyngées ou buccales du patient étant un indicateur d'un dysfonctionnement de la barrière cellulaire associé à un syndrome du côlon irritable (SCI) ou à une maladie inflammatoire intestinale (MII) chez le patient.

2. Méthode *in vitro* selon la revendication 1, dans laquelle ladite coloration est effectuée sur
- (i) des cellules épithéliales intestinales du patient obtenues du patient par biopsie ou aspiration, ou
- (i) des cellules épithéliales oropharyngées obtenues du patient par une biopsie dentaire, ou
- (i) des cellules épithéliales buccales obtenues du patient par frottis dans la joue.

3. Méthode *in vitro* selon la revendication 1, dans laquelle la sonde est un conjugué du tétrapeptide YVAD et d'un fluorochrome, et dans laquelle la sonde a en particulier la structure Alexa Fluor 488-GGGG-YVAD-FMK.

4. Méthode *in vitro* selon la revendication 1, comprenant en outre la coloration des cellules épithéliales intestinales, oropharyngées ou buccales avec une deuxième sonde détectable spécifique des caspases 3 et 7 et la détermination du ratio entre le marqueur associé à la caspase 1 et le marqueur associé aux caspases 3 et 7, la deuxième sonde étant en particulier un conjugué d'inhibiteur I des caspases 3/7 et d'un fluorochrome.

5. Méthode *in vitro* selon la revendication 4, dans laquelle le rapport entre les marqueurs de caspase 1 et de caspases 3 et 7 est significativement plus bas, en particulier plus bas d'au moins 40 %, chez les sujets en bonne santé que chez les sujets porteurs d'un SCI ou d'une MII.

6. Méthode *in vitro* selon la revendication 1, dans laquelle un taux élevé de caspase 1 est utilisé comme indication d'un traitement du patient par un inhibiteur de la caspase 1 ou un agent anti-inflammatoire.
